(19) Europäisches Patentamt
European Patent Office
Office européen des brevets

(11) Veröffentlichungsnummer: **0 140 362**
**B1**

(12) # EUROPÄISCHE PATENTSCHRIFT

(45) Veröffentlichungstag der Patentschrift: **22.07.87**

(51) Int. Cl.⁴: **C 07 C 149/36**, C 09 K 15/14, C 08 K 5/37

(21) Anmeldenummer: **84113021.4**

(22) Anmeldetag: **29.10.84**

(54) **Alkylierte S-(2-Hydroxyphenylthio)-carbonsäureester.**

(30) Priorität: **31.10.83 US 547520**

(43) Veröffentlichungstag der Anmeldung: **08.05.85 Patentblatt 85/19**

(45) Bekanntmachung des Hinweises auf die Patenterteilung: **22.07.87 Patentblatt 87/30**

(84) Benannte Vertragsstaaten: **DE FR GB IT**

(56) Entgegenhaltungen: **EP-A-0 079 855**
**DE-A-1 936 463**

(73) Patentinhaber: **CIBA- GEIGY AG, Klybeckstrasse 141, CH- 4002 Basel (CH)**

(72) Erfinder: **Spivack, John D., 1 Blue Jay Street, Spring Valley New York 10977 (US)**
Erfinder: **Pastor, Stephen D., 112 Union Road, Spring Valley New York 10977 (US)**
Erfinder: **Odorisio, Paul, 269 13th Street, Palisades Park New Jersey 07650 (US)**

(74) Vertreter: **Zumstein, Fritz, Dr., Bräuhausstrasse 4, D-8000 München 2 (DE)**

**EP 0 140 362 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Anmeldung betrifft neue alkylierte S-(2-Hydroxy-phenylthio)-carbonsäureester und deren Verwendung als Stabilisatoren für organisches Material.

Organische polymere Verbindungen wie Kunststoffe oder Harze unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophoren führen. Weiterhin müssen Probleme der Verarbeitungsstabilität und der Substratverträglichkeit beachtet werden.

Ueberraschenderweise ist jetzt gefunden worden, dass die alkylierten S-(2-Hydroxyphenylthio)-carbonsäureester dieser Erfindung eine ungewöhnliche Kombination wünschenswerter Eigenschaften besitzen, wodurch diese Stoffe zu besonders effektiven Stabilisatoren werden.

Die erfindungsgemässen Verbindungen erweisen sich als besonders nützliche Stabilisatoren für Polyolefine, schlagfestes Polystyrol, Elastomere wie Polybutadien oder Styrol-Butadien-Elastomere, bei denen der Erhalt der Elastizität und die Verhinderung von Vernetzungsreaktionen, von Versprödung, von Verfärbung, von Geruchsbildung und von Ausschwitzen des Stabilisators grundlegende Qualitätsanforderungen darstellen.

Eine Anzahl von p-Mercaptophenolderivaten ist bereits im Zusammenhang mit der Stabilisierung von organischem Material beschrieben worden, insbesondere S-(4-Hydroxyphenylthio)-carbonsäureester in EP-A-79 855, US Patent 3 989 644 und JP-A-76/142 097. Diese Verbindungen genügen jedoch nicht immer den heutigen Anforderungen der Technik.

Die vorliegende Erfindung betrifft Verbindungen der Formel I

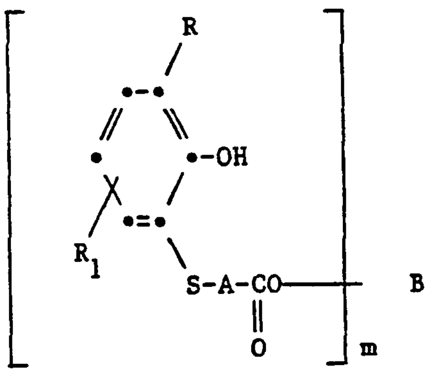

(I)

worin R $C_1$-$C_{18}$ Alkyl, $C_5$-$C_6$ Cycloalkyl, unsubstituiertes oder durch $C_1$-$C_{18}$ Alkyl substituiertes Phenyl, unsubstituiertes oder durch $C_1$-$C_{18}$ Alkyl substituiertes $C_7$-$C_9$ Aralkyl ist, $R_1$ Wasserstoff oder R bedeutet, A eine Gruppe $-CH(R_2)-$, $-CH(R_2)CH(R_3)-$ oder $-CH_2-CH(R_2)-CH_2-$ ist, $R_2$ und $R_3$ unabhängig voneinander Wasserstoff oder Methyl bedeuten, m eine Zahl 1 bis 4 ist,

B, wenn m = 1, $C_1$-$C_{18}$ Alkyl,

wenn m = 2, ununterbrochenes oder durch 1 oder 2 Sauerstoff-, -Schwefel- oder Stickstoffatome unterbrochenes $C_2$-$C_{10}$ Alkylen, $C_4$-$C_8$ Alkenylen, $C_4$-$C_8$ Alkinylen oder $C_5$-$C_6$ Cycloalkylen, und wenn m = 3-4, Alkanpolyyl mit 3 bis 6 Kohlenstoffatomen bedeutet.

Als $C_1$-$C_{18}$ Alkyl bedeuten R, $R_1$ und $R_2$ geradkettiges oder verzweigtes Alkyl, bevorzugt $C_1$-$C_8$ Alkyl, wie z. B. Methyl, Ethyl, Propyl, Isopropyl, n-Butyl, sek.-Butyl, tert.-Butyl, tert.-Pentyl, Hexyl, 2-Ethylhexyl, n-Octyl und 1,1,3,3-Tetramethylbutyl.

Bedeuten R und $R_1$ Aralkyl, dann sind sie z. B. Benzyl, α-Methylbenzyl oder α,α-Dimethylbenzyl, substituiertes Phenyl bedeutet z. B. Tolyl, Mesityl oder Xylyl.

Als $C_2$-$C_{10}$ Alkylen kann B geradkettiges oder verzweigtes Alkylen, vorzugsweise mit 2 bis 6 Kohlenstoffatomen sein, wie z. B. Ethylen, Propylen, Triethylen, Tetramethylen, 2,2-Dimethylpropan-1,3-diyl, Pentamethylen oder Hexamethylen. Als durch Sauerstoff oder Schwefel unterbrochenes Alkylen bedeutet B z. B. 3,6-Dioxaoctamethylen oder 3-Thiapentamethylen. Als durch Stickstoff unterbrochenes Alkylen bedeutet B z. B. N-Methyl-3-aza-pentamethylen.

B bedeutet als $C_4$-$C_8$ Alkenylen z. B. Hex-3-en-1,6-ylen oder bevorzugt But-2-en-1,4-ylen.

B bedeutet als $C_4$-$C_8$ Alkinylen z. B. Hex-3-in-1,6-ylen oder bevorzugt But-2-in-1,4-ylen.

Als Cycloalkylen ist B bevorzugt Cyclohexylen.

B ist als Alkanpolyyl beispielsweise eine Gruppe der Formel

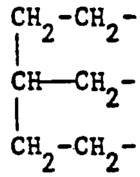

oder bevorzugt Pentaerythrityl.

Diejenigen Verbindungen der Formel I sind bevorzugt, worin R und $R_1$ verzweigtes $C_4$-$C_8$ Alkyl, insbesondere tert.-Butyl, sind und $R_1$ in p-Stellung zur OH-Gruppe steht.

A ist vorzugsweise eine Gruppe $-CH_2CH_2-$ oder $-CH_2-CH(CH_3)-$, wobei $-CH_2-$ am Schwefelatom gebunden ist. m ist bevorzugt 2 oder 4. B bedeutet insbesondere $C_1$-$C_8$ Alkyl, wenn m = 1 ist, $C_2$-$C_6$ Alkylen, 3,6-Dioxaoctamethylen oder 3-Thiapentamethylen, wenn m = 2 ist und Pentaerythrityl, wenn m = 4 ist.

Die erfindungsgemässen Verbindungen können hergestellt werden durch Umsetzung von ca. m Mol eines Thiocarbonsäureesters der Formel II

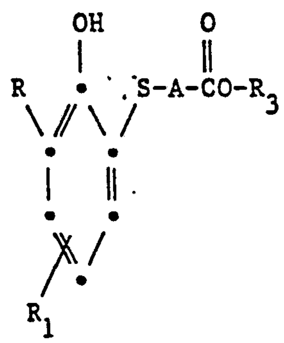

(II)

mit ca. 1 Mol eines Alkohol $B(OH)_m$, wobei $R_3$ Niederalkyl, insbesondere Methyl, bedeutet und m, R, $R_1$, A und B die oben angegebene Bedeutung haben, in Gegenwart eines Protonenakzeptors und anschliessendem Entfernen des entstandenen Niederalkylalkohols. Geeignete Protonenakzeptoren sind z. B. Lithiumsalze, tertiäre Amine, Alkalimetalle, Alkalimetall- und Erdalkalimetallhydroxide, Carbonate. Obwohl die Reaktion im allgemeinen in der Schmelze durchgeführt wird, ist es auch möglich in Gegenwart eines Lösungsmittels zu arbeiten. Das Lösungsmittel ist vorzugsweise aromatisch, wie z. B. Benzol, Toluol oder Xylol. Uebliche Reaktionstemperaturen sind im Bereich von 75 bis 200° C.

Ein weiteres Verfahren zur Herstellung der erfindungsgemässen Verbindungen besteht in der Umsetzung von ca. m Mol eines Mercaptophenols der Formel III

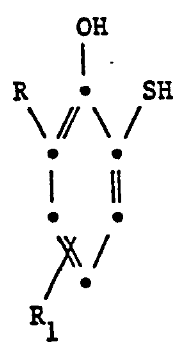

(III)

mit ca. 1 Mol eines Acrylats der Formel IV,

$$\left[CH_2=C(R_2)-\overset{\overset{\displaystyle O}{\|}}{C}O-\right]_m B \qquad (IV)$$

wobei m, R, $R_1$, $R_2$ und B die oben angegebene Bedeutung haben, in Gegenwart von je einem der oben erwähnten Lösungsmittel und Protonenakzeptoren.

Die zur Herstellung der erfindungsgemässen Verbindungen erforderlichen Ausgangsprodukte sind bekannt und im Handel erhältlich. Sollten einige von ihnen noch neu sein, so können sie in Analogie zu bekannten Verfahren hergestellt werden.

Geeignete Alkohole $B(OH)_m$ sind z. B. Pentaerythrit, 2,2-Dimethyl-1,3-propandiol, Triethylenglykol, 2,2'-Thiodiethanol-trimethylolpropan, 1,6-Hexandiol. Geeignete β-ungesättigte Ester der Formel IV sind z. B. Methylacrylat, Ethylacrylat, n-Propylacrylat, Butylmethacrylat, 2-Ethylhexylacrylat, n-Dodecylmethacrylat, n-Octadecylacrylat, n-Octadecylmethacrylat, Pentaerythrit-tetraacrylat, -1,6-Hexandiol-diacrylat, Trimethylolpropan-triacrylat, Sorbitolhexaacrylat, Mannitol-hexaacrylat, Clycerol-triacrylat, Ethylenglykoldiacrylat, Diethylenglykol-diacrylat, Triethylenglykoldiacrylat, Tetraethylenglykol-dimethacrylat.

Die erfindungsgemässen Verbindungen lassen sich als effektive Stabilisatoren für Kunststoffe, Polymere oder Harze einsetzen. Sie lassen sich auch als Stabilisatoren in Mineralölen oder synthetischen Ölen verwenden. Sie zeichnen sich insbesondere für ihre Wirksamkeit bezüglich Lichtschutz und Farbverhalten aus.

Die Erfindung betrifft daher auch eine Zusammensetzung enthaltend ein gegen oxidativen, thermischen oder strahlungsinduzierten Abbau empfindliches organisches Material und mindestens eine Verbindung der Formel I als Stabilisator, wobei als organisches Material vorzugsweise ein Polymer zu verstehen ist.

Vorzugsweise verwendet man die Verbindungen der Formel I als Stabilisatoren für Polyolefine wie beispielsweise Polyethylen oder Polypropylen; Polystyrol, vorzugsweise schlagfestes Polystyrol; ABS-Harz; Elastomere wie beispielsweise Polybutadien, EPM, EPDM, SBR, Nitril-Kautschuk oder natürlicher Kautschuk.

Polymere, die mit den erfindungsgemässen Verbindungen stabilisiert werden können, sind beispielsweise:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z. B. einem Polyacrylat, einem Dien-Polymeren oder einem Ethylen-Propylen-Dien-Terpolymeren; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z. B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat,

Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenyloxide und -sulfide und deren Mischungen mit Styrol-Polymeren.

14. Polyurethane, die sich von Polyethern, Polyestern und Polybutadienen mit endständigen Hydroxylgruppen einerseits und aliphstischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexa-methylenterephthalamid, Poly-m-phenylenisophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z. B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z. B. von Bis-glycidylethern oder von cycloaliphstischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z. B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z. B. Phthalate, Adipate, Phosphate oder Trimellithete), sowie Abmischungen synthetischer Ester mit Mineralölen in beliebigen Gewichtsverhältnissen, wie sie z. B. als Spinnpräparationen Anwendung finden, sowie deren wässrigen Emulsionen.

29. Wässrige Emulsionen natürlicher oder synthetischer Kautschuke, wie z. B. Naturkautschuk-Latex oder Latices von carboxylierten Styrol-Butadien-Copolymeren.

Die erfindungsgemässen Stabilisatoren werden den Kunststoffen in Mengen von 0,01 bis 5 Gew.-% bezogen auf das stabilisierte Material zugemischt. Je nach Verwendung und Substrat wird die Stabilisatormenge variiert. Bevorzugt verwendet man 0,05 bis 2 Gew.-% des Stabilisators, besonders bevorzugt 0,1 bis 1 Gew.-%.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit den pulverförmigen Polymeren gemischt werden, oder eine Emulsion bzw. eine Suspension des Stabilisators kann mit einer Lösung, Suspension oder Emulsion des Polymeren vermischt werden.

Die so stabilisierten Materialien können in verschiedenster Form angewendet werden, z. B. als Folien, Fasern, Bändchen, Formmassen, Profile oder als Bindemittel für Lacke, Klebstoffe oder Kitte.

In der Praxis können die Verbindungen der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z. B. weitere aminische Antioxidantien, Metallpassivatoren, Rostinhibitoren, Viskositäts-Index Verbesserer, Stockpunkterniedriger, Dispergiermittel/Tenside und Verschleissschutzadditive. Als weitere Additive, mit denen zusammen die erfindungsgemäss verwendeten Stabilisatoren eingesetzt werden können, sind beispielsweise zu nennen:

## 1. Antioxidantien

### 1.1. Alkylierte Monophenole

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

### 1.2. Al-kylierte Hydrochinone

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

### 1.3. Hydroxylierte Thiodiphenylether

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

### 1.4. Alkyliden-Bisphenole

2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercapto-butan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

### 1.5 Benzylverbindungen

1,3,5-Tri-(3,5-di-tert.butyl-4-hydroxybenzyl)-2,4,6-trimethylbenzol Di-(3,5-di-tert.butyl-4-hydroxybenzyl)-sulfid
3,5-di-tert.butyl-4-hydroxybenzyl-mercaptoessigsäure-isooctylester Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat

3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

**1.6. Acylaminophenole**

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

**1.7. Ester der ß-(3,5-Di-tert.butyl-4-hydroxyphenyl)-propionsäure**

mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

**1.8. Ester der ß-(5-tert.butyl-4-hydroxy-3-methylphenyl)-propion säure** mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

**1.9. Amide der ß-(3,5-Di-tert.butyl-4-hydroxyphenyl)-propionsäure,**

wie z. B.

N,N'-Di-(3,5-di-tert.butyl-4-hydroxyphenylpropionyl)-hexamethylen-diamin
N,N'-Di-(3,5-di-tert.butyl-4-hydroxyphenylpropionyl)-trimethylen-diamin
N,N'-Di-(3,5-di-tert.butyl-4-hydroxyphenylpropionyl)-hydrazin

**2. UV-Absorber und Lichtschutzmittel**

**2.1. 2-(2'-Hydroxyphenyl)-benztriazole**, wie z. B. das 5'-Methyl-, 3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Tri-hydroxy-, 2'-Hydroxy-4,4'-dimethoxy-Derivat.

2.3. Ester von gegebenenfalls substituierten Benzoesäuren, wie z. B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

2.5. Nickelverbindungen, wie z. B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin

oder N-Cyclohexyl-diethanolamin, Nickeldibutyldithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-unde-cylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z. B. Bis-(2,2,6,6-tetramethyl-piperidyl)-sebacat Bis-(1,2,2,6,6-pentamethylpiperidyl)-sebacat n-Butyl-3,5-di-tert.butyl-4-hydroxybenzyl-malonsäure-bis(1,2,2,6,6-pentamethylpiperidyl)-ester, Kondensationsprodukt aus 1-Hydroxy-ethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-tri-azin,
Tris-(2,2,6,6-tetramethyl-4-piperidyl)-nitrilotriacetat,
Tetrakia-(2,2,6,6-tetramethyl-4-piperidyl)-1,2,3,4-butantetracarbon-säure,
1,1'-(1,2-Ethandiyl)-bis-(3,3,5,5-tetramethyl-piperazinon).

2.7. Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.bu-tylphenyl)-4,4'-biphenylen-diphosphonit.

5. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercapto-benzimidazols, Zink-dibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Jodiden und/oder Phosphorverbindungen und Salze des zweiwertigen Mangans.

7. Basische Co-Stabilisatoren, wie z. B. Melamin, Polyvinylpyrrolidon, Dicyandiamid, Triallylcyanurat, Harnstoff-Drivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert.Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel.

Für den Einsatz zusammen mit den erfindungsgemässen Verbindungen bevorzugte Costabilisatoren sind Antioxidantien, Lichtschutzmittel, Metalldesaktivatoren, Phosphite, Phosphonite und Thiosynergisten (peroxidzerstörende Verbindungen).

Die folgenden Beispiele erläutern die Erfindung. Alle Mengenangaben entsprechen Gewichtsteilen soweit nicht anderweitig angegeben.

**Beispiel 1: 3-(3,5-Di-tert.-butyl-2-hydroxyphenylthio)-propionsäure-methylester**

In einem 50 ml Kolben wird unter Stickstoffzuleitung ein Gemisch aus 5,0 g 2,4-Di-tert.-butyl-6-mercaptophenol, 1,8 g Methylacrylac und 20 ml Toluol mit 0,5 ml Triethylamin zur Reaktion gebracht. Nach 3 Stunden Rühren bei Zimmertemperatur wird das Lösungsmittel im Vakuum abdestilliert. Der Rückstand wird chromatographisch gereinigt (auf Silicagel mit Toluol/Heptan als Elutionsmittel). Man erhält 3,9 g (57 % der Theorie) eines farblosen Öls.

Analyse für $C_{18}H_{28}O_3S$
Berechnet: S 9,9
Gefunden: S 10,1

**Beispiel 2:, 3-(3,5-Di-tert.-butyl-2-hydroxyphenylthio)-propionsäure(2-ethylhexyl)-ester**

Das Verfahren von Beispiel 1 wird wiederholt mit 20,0 g 2,4-Di-tert.-butyl-6-mercaptophenol, 15,5 g 2-Ethylhexyl-acrylat, 0,25 ml Triethylamin und 150 ml Toluol. Der Rückstand wird chromatographisch gereinigt (auf Silicagel mit Toluol/Heptan als Elutionsmittel). Man erhält 12,4 g (35 % d.Th.) eines farblosen Öls.

Analyse für $C_{25}H_{42}O_3S$

Berechnet: C 71,0, H 10,0, S 7,6
Gefunden : C 71,4, H 10,4, S 7,4

**Beispiel 3: Bis[3-(3,5-di-tert.-butyl-2-hydroxyphenylthio)-2-methylpropionsäure]-triethylglycol-diester**

In einem 200 ml Kolben wird unter Stickstoffzuleitung eine Lösung von 23,8 g 2,4-Di-tert.-butyl-6-mercaptophenol in 50 ml Toluol mit 0,04 g Lithiumhydrid versetzt. Nach 30 Minuten Rühren werden 14,3 g Triethylenglycol-dimethylacrylat zugegeben. Die Mischung wird dann auf 80°C erhitzt bis nach dünnschichtchromatographischer Analyse alle Reaktanden verbraucht sind (ca. 2 Stunden). Das Reaktionsgemisch wird dann mit 10 ml 1N wässriger Salzsäure behandelt und die organische Schicht abgetrennt, mit Natriumsulfat getrocknet und im Vakuum eingeengt. Der Rückstand wird chromatographisch gereinigt (auf Silicagel mit Essigsäureethylester/Methylenchlorid als Elutionsmittel). Man erhält 10,0 g (26 % d.Th.) einer farblosen viskosen Flüssigkeit.

Analyse für $C_{42}H_{66}O_8S_2$
Berechnet: C 66,1, H 8,7, S 8,4
Gefunden : C 66,0, H 8,3, S 8,6

**Beispiel 4: Tetrakis-[3-(3,5-di-tert.-butyl-2-hydroxyphenylthio) -propionsäure]-pentaerythrit-tetraester**

In einem mit Dean Stark-Falle versehenem 100 ml Kolben wird ein Gemisch aus 30,0 g der Verbindung von Beispiel 1, 3, 15 g Pentaerythrit und 0,02 g Lithiumhydrid 11 Stunden bei einem Druck von 65 mbar auf 140-165°C erhitzt. Der Rückstand wird chromatographisch gereinigt (auf Silicagel mit Essigsäureethylester/Heptan als Elutionsmittel). Man erhält 16,0 g (53 % d.Th.) einer farblosen festen Substanz mit Smp. 124-127°C.

Analyse für $C_{73}H_{108}O_{12}S_4$

| | | | |
|---|---|---|---|
| Berechnet: | C 67,1, | H 8,3, | S 9,8 |
| Gefunden : | C 67,3, | H 8,4, | S 9,5 |

**Beispiel 5: Bis-[3-(3,5-di-tert.-butyl-2-hydroxyphenylthio)-propion säure]-2,2'-thiodiethyl-diester**

Das Verfahren von Beispiel 4 wird wiederholt mit 30,0 g der Verbindung von Beispiel 1, 5,64 g 2,2'-Thiodiethanol und 0,02 g Lithiumhydrid. Der Rückstand wird chromatographisch gereinigt (auf Silicagel mit Toluol/Essigsäureethylester als Elutionsmittel). Man erhält 22,0 g (67 % d.Th.) einer farblosen festen Substanz mit Smp. 64-66°C.

Analyse für $C_{38}H_{58}O_6S_3$

| | |
|---|---|
| Berechnet: | S 13,6 |
| Gefunden : | S 13,2 |

**Beispiel 6: Stabilisierung von Polypropylen** (Lichtstabilität)

Unstabilisiertes Polypropylenpulver (Hercules Profax® 6501) wird mit 0,2 Gew.-% eines Additives gemischt. Die Mischung wird anschliessend bei 182°C 5 Minuten lang auf einem Mischwalzwerk plastifiziert. Anschliessend wird die stabilisierte Polypropylenfolie vom Walzwerk abgelöst und abkühlen gelassen. Die Folie wird in Stücke geschnitten und auf einer hydraulischen Presse bei 220°C und 12 bar zu einer 0,13 mm dicken Probe verformt. Diese Probe wird ultraviolettem Licht bis zur Zersetzung ausgesetzt. Als Zersetzungszeitpunkt wird die Zeit in Stunden angegeben bei der eine Carbonylabsorption von 0,5 (IR-Spektroskopie) erreicht wird. Die Messdaten sind in der folgenden Tabelle 1 dargestellt:

**Tabelle 1:**

| Additiv (Produkt von Beispiel) | Zahl der Stunden bis zur Zersetzung der bestrahlten Proben |
|---|---|
| keines | 250 |
| 2 | 420 |
| 3 | 370 |
| 4 | 370 |
| 5 | 400 |

**Beispiel 7: Stabilisierung von Polypropylen** (Oxidationsstabilität)

Um die Oxidationsstabilität von Polypropylen, das 0,2 Gew. % eines erfindungsgemässen Additives oder einer synergistisch wirkenden Formulierung bestehend aus 0,1 Gew.-% eines Additivs und 0,3 Gew.-% Distearyldithiodipropionat (DSTDP) enthält, zu untersuchen, werden Plättchen von 0,64 mm Dicke hergestellt und in einem Umluftofen bei 150°C gealtert. Als Zersetzungszeitpunkt wird diejenige Anzahl von Stunden gewertet, nach der die Probe die ersten Zerfallserscheinungen zeigt (braune Ecken, Risse).

Die Messdaten sind in Tabelle 2 dargestellt:

**Tabelle 2**

| Additiv (Produkt von Beispiel) | Stunden bis zur Zersetzung |
|---|---|
| keines | < 20 |
| 0,3 % DSTDP | < 20 |
| 0,2 % 3 | 70 |
| 0,2 % 4 | 100 |
| 0,1 % 4 + 0,3 % DSTDP | 140 |
| 0,2 % 5 | 110 |

**Beispiel 8: Stabilisierung von schlagfestem Polystyrol**

Man stellt eine Lösung von 8 Gew.-% Polybutadien-Kautschuk (Fireston DIENE 55®) in monomerem Styrol her, indem man beide Komponenten in einer Quetschmühle mischt. Gleichzeitig werden in diesem Arbeitsgang 0,1 Gew.-% des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, dass mit einem Wendelrührer ausgerüstet ist. Da die meisten IPS Blockpolymerisationen thermisch initiiert werden, wird in der vorliegenden Laboratoriumversion kein Starter zugesetzt. Die Reaktion wird unter Stickstoff und unter Erwärmen durchgeführt. Der Reaktor wird innerhalb einer halben Stunde auf 121°C erwärmt, und diese Temperatur wird aufrecht erhalten, bis zwischen 30 und 35 % des Monomeren umgesetzt sind (Zeitdauer ca. 2,5 Stunden). Während der Polymerisation wird heftig gerührt, und die Rührgeschwindigkeit wird so eingestellt, dass Polymerpartikel von 2 bis 4 µm Grösse entstehen. Anschliessend werden die Reaktionsgefässe dem Reaktor entnommen, im Stickstoffstrom geöffnet und in ein Wirbelschichtsandbad gestellt, um die Polymerisation zu vervollständigen. Die Gefässe werden im Sandbad folgendermassen erhitzt: eine Stunde bei 100°C um die Anfangstemperatur vorzugeben, eine weitere Stunde um 140°C zu erreichen und anschliessend weitere 8 Stunden, wobei die Temperatur jede Stunde um 10°C erhöht wird, bis schliesslich ein Maximum von 220°C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200°C für 45 Minuten bei einem Druck von 1,33 mbar belassen, um flüchtige Bestandteile zu entfernen. Nach dieser Behandlung wird der Block sofort in einer heizbaren hydraulischen Presse hei 205°C zwischen zwei Aluminiumfolien zu einem dicken Stab gepresst (drei Minuten Erhitzen, fünf Minuten ungeheizt).

Der Stab wird mittels einer Bandsäge zerkleinert und anschliessend granuliert. Alle Polymermischungen werden bei 205°C extrudiert und dann zerkleinert. Die Pellets werden unter Druck bei 205°C in dehnbare 3,2 mm dicke Streifen verformt. Diese Probestreifen werden dann bei 150°C in einem Umluftofen gealtert, wobei sie auf Glasplatten, die sich wiederum auf rotierenden Trägern befinden, aufgebracht sind. Nach bestimmten Zeitintervallen wird der Yellowness Index der Proben gemäss ASTM D-1925-63T bestimmt.

Die Resultate sind in Tabelle 3 dargestellt:

**Tabelle 3:**

| Additiv (Produkt von Beispiel) | Yellowness Index nach Stunden bei 150°C | | | | |
|---|---|---|---|---|---|
| | 0 h | 0,5 h | 1 h | 1,5 h | 2 h |
| keines | 7 | 18 | 30 | 38 | 43 |
| 4 | -2 | 0 | 4 | 4 | 15 |

**Patentansprüche**

1. Verbindungen der Formel I

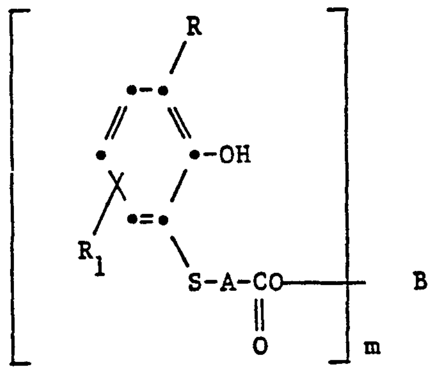

(I)

worin R $C_1$-$C_{18}$ Alkyl, $C_5$-$C_6$ Cycloalkyl, unsubstituiertes oder durch $C_1$-$C_{18}$ Alkyl substituiertes Phenyl, unsubstituiertes oder durch $C_1$-$C_{18}$ Alkyl substituiertes $C_7$-$C_9$ Aralkyl ist, $R_1$ Wasserstoff oder R bedeutet, A eine Gruppe $-CH(R_2)-$, $-CH(R_2)CH(R_3)-$ oder
$-CH_2-CH(R_2)-CH_2$ - ist, $R_2$ und $R_3$ unabhängig voneinander Wasserstoff oder Methyl bedeuten, m eine Zahl 1 bis 4 ist,
B, wenn m = 1, $C_1$-$C_{18}$ Alkyl,
wenn m = 2, ununterbrochenes oder durch 1 oder 2 Sauerstoff-, Schwefel- oder Stickstoffatome unterbrochenes $C_2$-$C_{10}$ Alkylen, $C_4$-$C_8$ Alkenylen, $C_4$-$C_8$ Alkinylen oder $C_5$-$C_6$ Cycloalkylen, und wenn m = 3-4, Alkanpolyyl mit 3 bis 6 Kohlenstoffatomen bedeutet.

2. Verbindungen der Formel 1, gemäss Anspruch 1, dadurch gekennzeichnet, dass R und $R_1$ verzweigtes $C_4$-$G_8$ Alkyl bedeuten.

3. Verbindungen der Formel I, gemäss Anspruch 2, dadurch gekennzeichnet, dass $R_1$ in p-Stellung zur OH-Gruppe steht.

4. Verbindungen der Formel I, gemäss Anspruch 3, dadurch gekennzeichnet, dass R und $R_1$ tert.-Butyl bedeuten.

5. Verbindungen der Formel I, gemäss Anspruch 1, dadurch gekennzeichnet, dass A eine Gruppe $-CH_2CH_2-$ oder $-CH_2CH(CH_3)-$ ist, wobei $-CH_2-$ am Schwefelatom gebunden ist.

6. Verbindungen der Formel I, gemäss Anspruch 1, dadurch gekennzeichnet, dass m = 1 und B $C_1$-$C_8$ Alkyl sind.

7. Verbindungen der Formel I, gemäss Anspruch 1, dadurch gekennzeichnet, dass m die Zahlen 2 oder 4 bedeutet.

8. Verbindungen der Formel I, gemäss Anspruch 7, dadurch gekennzeichnet, dass,
wenn m = 2, B $C_2$-$C_6$ Alkylen, 3,6-Dioxaoctamethylen oder 3-Thia-pentamethylen und
wenn m = 4, B Pentaerythrityl bedeutet.

9. Stoffzusammensetzung enthaltend ein gegen oxidativen, thermischen oder strahlungsinduzierten Abbau empfindliches organisches Material und mindestens eine Verbindung der Formel I gemäss Anspruch 1 als Stabilisator.

10. Stoffzusammensetzung gemäss Anspruch 9, worin unter dem organischen Material ein synthetisches Polymer zu verstehen ist.

11. Stoffzusammensetzung gemäss Anspruch 9, worin unter dem organischen Material ein Polyolefin, schlagfestes Polystyrol, ABS-Harz, Polybutadien, EPM, EPDM, SBR, Nitrilkautschuk oder natürlicher Kautschuk zu verstehen ist.

12. Stoffzusammensetzung gemäss Anspruch 9, enthaltend zusätzlich mindestens einen Costabilisator ausgewählt aus der Gruppe bestehend aus Antioxidantien, Lichtschutzmittel, Metalldesaktivatoren, Phosphite, Phosphonite und Thiosynergisten.

**Claims**

1. A compound of the formula

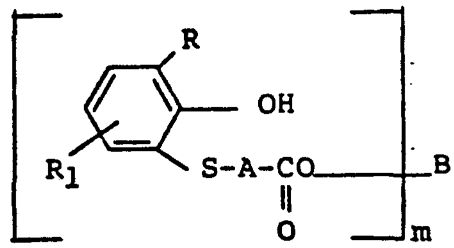

(I)

wherein R is alkyl of 1 to 18 carbon atoms, cycloalkyl of 5 to 6 carbon atoms, phenyl, phenyl substituted by alkyl of 1 to 18 carbon atoms, or aralkyl of 7 to 9 carbon atoms or said aralkyl substituted by alkyl of 1 to 18 carbon atoms;

$R_1$ is hydrogen or R,

A is $-CH(R_2)-$, $-CH(R_2)CH(R_3)-$or $-CH_2-CH(R_2)-CH_2-$;

$R_2$ and $R_3$ are independently hydrogen or methyl;

m is 1 to 4;

when m = 1, B is alkyl of 1 to 18 carbon atoms;

when m = 2, B is alkylene of 2 to 10 carbon atoms,

which alkylene group may be interrupted by one or two oxygen, sulfur or nitrogen atoms, or alkenylene of 4 to 8 carbon atoms, alkynylene of 4 to 8 carbon atoms or cycloalkylene of 5 to 6 carbon atoms; and when m = 3-4, B is an alkane polyyl of 3 to 6 carbon atoms.

2. A compound of formula I according to claim 1, wherein R and $R_1$ are branched alkyl of 4 to 8 carbon atoms.

3. A compound of formula I according to claim 2, wherein $R_1$ is in the para position to the hydroxy group.

4. A compound of formula I according to claim 3, wherein R and $R_1$ are tert-butyl.

5. A compound of formula I according to claim 1, wherein A is $-CH_2CH_2-$ or $-CH_2CH(CH_3)-$, with $-CH_2-$ being attached to S.

6. A compound of formula I according to claim 1, wherein m is 1 and B is alkyl of 1 to 8 carbon atoms.

7. A compound of formula I according to claim 1, wherein m is 2 or 4.

8. A compound of formula I according to claim 7, wherein when m = 2, B is alkylene of 2 to 6 carbon atoms, 3,6-dioxaoctamethylene or 3-thiapentamethylene and when m = 4, B is pentaerythrityl.

9. A composition comprising an organic material subject to oxidative, thermal and radiation-induced degradation stabilized with at least one compound of formula I according to claim 1.

10. A composition according to claim 9, wherein the organic material is a synthetic polymer.

11. A composition according to claim 9, wherein said organic material is selected from the group consisting of polyolefins, impact polystyrene, ABS resin, polybutadiene, EPM, EPDM, SBR, nitrile rubber or natural rubber.

12. A composition according to claim 9, which additionally contains a costabiliser selected from the group consisting of antioxidants, light stabilisers, metal passivators, phosphites, phosphonites and thiosynergists.

**Revendications**

1. Composés répondant à la formule 1:

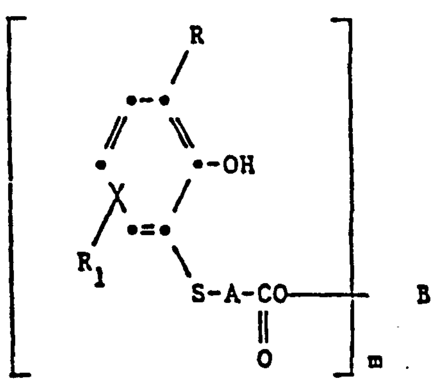

(I)

dans laquelle :

R représente un alkyle en $C_1$-$C_{18}$, un cycloalkyle en $C_5$ ou $C_6$, un phényle non substitué ou porteur d'un alkyle en $C_1$-$C_{18}$, ou un aralkyle en $C_7$-$C_9$ non substitué ou porteur d'un alkyle en $C_1$-$C_{18}$,

$R_1$ représente l'hydrogène ou a l'une des significations qui viennent d'être indiquées pour R,
A représente un radical $-CH(R_2)-$, $-CH(R_2)CH(R_3)-$ ou $-CH_2-CH(R_2)-CH_2-$, les symboles $R_2$ et $R_3$ représentant chacun, indépendamment l'un de l'autre, l'hydrogène ou un méthyle,
m désigne un nombre de 1 à 4 et
B représente:
- lorsque m est égal à 1, un alkyle en $C_1$- $C_{18}$,
- lorsque m est égal à 2, un alkylène en $C_2$-$C_{10}$ non interrompu ou interrompu par 1 ou 2 atomes d'oxygène, de soufre ou d'azote, un alcénylène en $C_4$-$C_8$, un alcynylene en $C_4$-$C_8$ ou un cycloalkylène en $C_5$ ou $C_6$ et
- lorsque m est égal à 3 ou à 4, un alcanepolyyle contenant de 3 à 6 atomes de carbone.

2. Composés de formule I selon la revendication 1 caractérisés en ce que R et $R_1$ représentent chacun un alkyle contenant de 4 à 8 atomes de carbone.

3. Composés de formule I selon la revendication 2 caractérisés en ce que $R_1$ se trouve en position para relativement au radical -OH.

4. Composés de formule I selon la revendication 3 caractérisés en ce que R et $R_1$ représentent chacun un radical tert-butyle.

5. Composés de formule I selon la revendication 1 caractérisés en ce que A représente un radical $-CH_2CH_2-$ ou un radical $-CH_2CH(CH_3)-$ relié à l'atome de soufre par son radical $-CH_2-$.

6. Composés de formule I selon la revendication 1 caractérisés en ce que m est égal à 1 et B représente un alkyle contenant de 1 à 8 atomes de carbone.

7. Composés de formule I selon la revendication 1 caractérisés en ce que m est égal à 2 ou à 4.

8. Composés de formule I selon la revendication 7 caractérisés en ce que, lorsque m est égal à 2, B représente un radical alkylène contenant de 2 à 6 atomes de carbone, un radical dioxa-3,6 octaméthylène ou un radical thia-3 pentaméthylène, et, lorsque m est égal à 4, B représente un radical pentaérythryle.

9. Composition contenant une matière organique susceptible de subir une dégradation sous l'action de l'oxydation, de la chaleur ou de la lumière et, comme stabilisant, au moins un composé de formule I selon la revendication 1.

10. Composition selon la revendication 9 qui contient, comme matière organique, un polymère synthétique.

11. Composition selon la revendication 9 qui contient, comme matière organique, une polyoléfine, un polystyrène choc, une résine ABS, un polybutadiène, de l'EPM, de l'EPDM, du SBR, du caoutchouc nitrile ou du caoutchouc naturel.

12. Composition selon la revendication 9 qui contient en outre au moins un co-stabilisant pris dans l'ensemble constitué par les anti-oxydants, les stabilisants à la lumière, les désactivants de métaux, les phosphites, les phosphonites et les agents de synergie sulfurés.